(19) Europäisches Patentamt
European Patent Office
Office européen des brevets

(11) Veröffentlichungsnummer: **0 107 123**
**B1**

(12) # EUROPÄISCHE PATENTSCHRIFT

(45) Veröffentlichungstag der Patentschrift: **01.07.87**

(21) Anmeldenummer: **83109953.6**

(22) Anmeldetag: **05.10.83**

(51) Int. Cl.⁴: **C 07 D 263/58**, C 07 D 277/68, C 07 D 277/74, A 01 N 47/30, A 01 N 47/20, A 01 N 43/78

(54) **Anilinderivate, Verfahren zu ihrer Herstellung und ihre Verwendung zur Bekämpfung unerwünschten Pflanzenwuchses.**

(30) Priorität: **14.10.82 DE 3238079**

(43) Veröffentlichungstag der Anmeldung: **02.05.84 Patentblatt 84/18**

(45) Bekanntmachung des Hinweises auf die Patenterteilung: **01.07.87 Patentblatt 87/27**

(84) Benannte Vertragsstaaten: **BE DE FR GB IT SE**

(56) Entgegenhaltungen:
**EP-A-0 035 712**
**EP-A-0 036 390**
**EP-A-0 044 497**
**DE-A-2 640 730**
**GB-A-1 447 428**

**CHEMICAL ABSTRACTS, Band 98, Nr. 23, 6. Juni 1983, Seite 654, Nr. 198207n, Columbus, Ohio, US**

***Die Akte enthält technische Angaben, die nach dem Eingang der Anmeldung eingereicht wurden und die nicht in dieser Patentschrift enthalten sind.***

(73) Patentinhaber: **BASF Aktiengesellschaft, Carl-Bosch- Strasse 38, D-6700 Ludwigshafen (DE)**

(72) Erfinder: **Becker, Rainer, Dr., Im Haseneck 22, D-6702 Bad Duerkheim (DE)**
Erfinder: **Spiegler, Wolfgang, Dr., Westpreussenstrasse 5, D-6520 Worms 27 (DE)**
Erfinder: **Schirmer, Ulrich, Dr., Berghalde 79, D-6900 Heidelberg (DE)**
Erfinder: **Wuerzer, Bruno, Dr. Dipl.- Landwirt, Ruedigerstrasse 13, D-6701 Otterstadt (DE)**

EP 0 107 123 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) *Europäisches Patentübereinkommen*).

LIBER, STOCKHOLM 1987

**Beschreibung**

Die Erfindung betrifft Anilinderivate, Verfahren sowie Zwischenprodukte zu ihrer Herstellung, Herbizide, die diese Verbindungen als Wirkstoffe enthalten, sowie ein Verfahren zur Bekämpfung unerwunschten Pflanzenwuchses mit diesen Verbindungen.

Es ist bekannt, daß Phenoxyphenylharnstoffe, N-Phenoxyphenyl-carbamate, N-Alkylcarbaminsäurephenylester, N-Alkyl-carbaminsäure-benzthiazolyloxyphenoxyalkylester sowie Benzthiazolyloxyphenoxy-alkancarbonsäurederivate herbizid wirksam sind (EP-A-0 036 390; EP-A-0 035 712; GB-A-1 447 428; DE-A-2 640 730; EP-A-0 044 497).

Es wurde gefunden daß Anilinderivate der Formel (I)

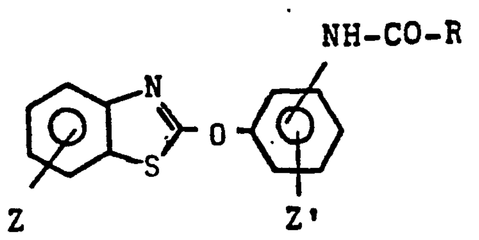

in der
Z Wasserstoff, $C_1$-$C_4$-Halogenalkyl oder Halogen,
Z' Wasserstoff, Halogen oder Trifluormethyl und
R $C_1$-$C_5$-Alkyl, $C_1$-$C_4$-Alkoxy, $C_1$-$C_4$-Alkylthio, gegebenenfalls durch $C_1$-$C_4$-Alkyl substituiertes $C_3$-$C_7$-Cycloalkyl, Di-$C_1$-$C_4$-alkylamino oder N-$C_1$-$C_4$-Alkyl-N-$C_1$-$C_4$-alkoxyamino bedeuten,
eine selektive herbizide Wirkung haben.

In der Formel (I) bedeutet Z Wasserstoff, $C_1$-$C_4$-Halogenalkyl, wie Trifluormethyl oder Difluormethyl, oder Halogen, wie Fluor, Chlor, Brom. Z' bedeutet neben Wasserstoff Trifluormethyl oder Halogen, wie Fluor, Chlor, Brom.

R bedeutet $C_1$-$C_5$-Alkyl, wie Methyl, Ethyl, Propyl Butyl, iso-Propyl, oder gegebenenfalls durch $C_1$-$C_4$-Alkyl, vorzugsweise Methyl, substituiertes $C_3$-$C_7$-Cycloalkyl, wie Cyclopropyl, 1-Methylcyclopropyl, oder $C_1$-$C_4$-Alkoxy, wie Methoxy, Ethoxy, oder $C_1$-$C_4$-Alkylthio, wie Methylthio, Ethylthio, oder N,N-Dialkylamino mit $C_1$-$C_4$-Alkylgruppen, wie Dimethylamino, oder N-Alkyl-N-alkoxyamino mit $C_1$-$C_4$-Alkyl und $C_1$-$C_4$ Alkoxygruppen, wie Methylmethoxyamino, Methylethoxyamino.

Bevorzugte Verbindungen der Formel (I) sind solche, bei denen die Gruppe -NH-CO-R in 4-Stellung steht und R N-$C_1$-$C_4$-Alkyl-N-$C_1$-$C_4$-alkoxyamino, insbesondere N-Methoxy-N-methylamino, bedeutet. Bevorzugter Substituent für Z ist Halogen, für Z' Wasserstoff oder Halogen.

Man erhält die Anilinderivate der Formel (I) durch Umsetzung von Verbindungen der Formel (II)

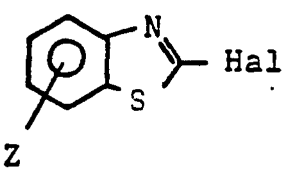

in der X und Z die oben genannten Bedeutungen haben und Hal für Halogen, insbesondere Chlor, steht, mit einem Phenol der Formel (III)

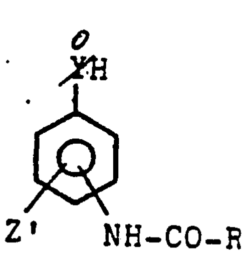

in der X, Z' und R die oben genannten Bedeutungen haben, bei einer Temperatur zwischen 20 und 150°C, vorzugsweise zwischen 50 und 120°C, in Gegenwart eines inerten Lösungsmittels und einer Base.

Die Menge an Base beträgt 1 bis 1,5 Mol pro Mol Verbindung (II). Geeignete Basen sind Alkalicarbonate, Alkalihydrogencarbonate, Alkalihydroxide, wie Kaliumcarbonat, Natriumcarbonat, Natriumhydroxid oder Kaliumhydroxid. Auch organische Basen, wie Triethylamin oder Pyridin, können Verwendung finden.

Als Lösungsmittel kommen polare organische Lösungsmittel, wie Aceton, Acetonitril, Dimethylformamid, Dimethylsulfoxid, sowie Wasser in Betracht.

Wird die Reaktion in wäßrigem Medium durchgeführt, so wird zweckmäßigerweise ein Phasentransferkatalysator, z.B. Tetrabutylammoniumjodid, zugesetzt. Die Katalysatormenge beträgt etwa 0,01 bis 0,1 Mol, bezogen auf 1 Mol der Verbindung der Formel (II).

Die Reaktionskomponenten werden in etwa äquimolarem Verhältnis umgesetzt. Ein Überschuß der einen oder anderen Verbindung stört nicht.

Weiterhin erhält man die Verbindungen der Formel (I) durch Umsetzung einer Verbindung der Formel (IV)

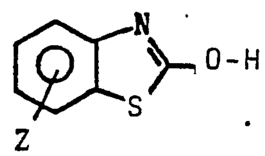

in der Z die oben genannten Bedeutungen hat, mit einem Halogennitrobenzolderivat der Formel (V)

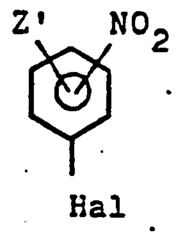

in der Z′ die oben genannten Bedeutungen hat und Hal für Halogen, vorzugsweise Fluor oder Chlor steht, Reduktion der so erhaltenen Nitroverbindung zum Amin der Formel (VI) und Umsetzung dieses Amins mit einer Verbindung der Formel RCOCl, in der R die oben genannten Bedeutungen hat:

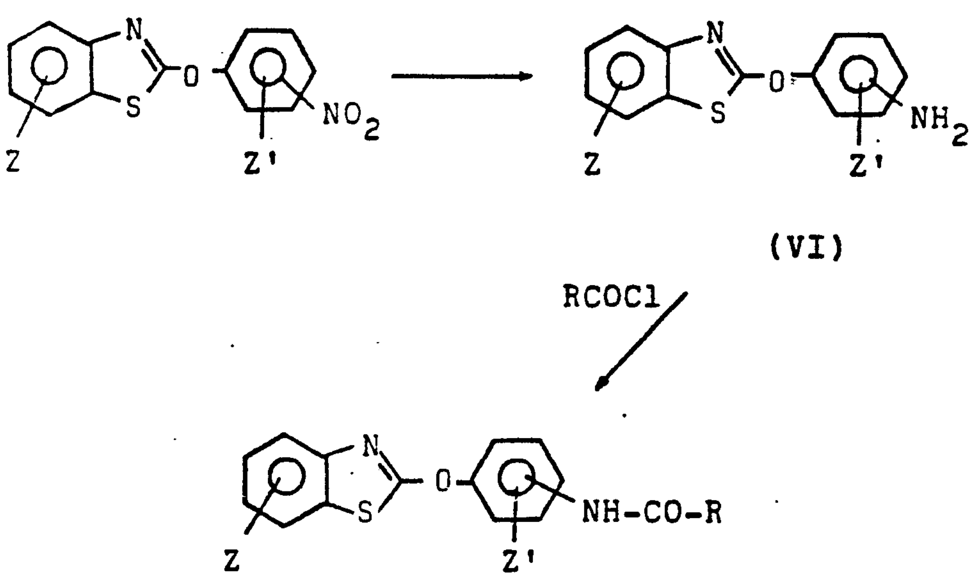


(VI)

(I)

Die Kondensation der Verbindungen der Formel (IV) mit den Verbindungen der Formel (V) verläuft unter den für die Umsetzung der Verbindungen der Formel (II) mit den Verbindungen der Formel (III) beschriebenen Bedingungen, d.h. in Gegenwart eines Lösungsmittels und einer Base. Die Reduktion der Nitroverbindung zum Amin der Formel (VI) kann beispielsweise in an sich üblicher Weise katalytisch mit Raney-Nickel oder Pd/C bzw. mit Eisenpulver oder Zinnchlorid erfolgen.

Die Umsetzung der Amine der Formel (VI) mit Verbindungen der Formel RCOCl verläuft bei Temperaturen zwischen 0 und 50°C in inerten Lösungsmitteln, wie Aceton, Acetonitril, Essigester, Tetrahydrofuran und in

Gegenwart von 1 bis 1,5 Moläquivalenten einer Base. Geeignete Basen sind Alkalicarbonate, Alkalihydrogencarbonate oder Hydroxide, wie Natriumcarbonat, Natriumhydrogencarbonat, Kaliumcarbonat, Natriumhydroxid, oder organische Basen, wie tertiäre Amine, z.B. Pyridin der Triethylamin.

Ebenso lassen sich die Anilinderivate der Formel (I) durch Umsetzung der Verbindung der Formel (II) mit einem Nitrophenol der Formel (VII) oder einem Aminophenol der Formel (VIII) unter den für die Reaktion von Verbindungen der Formel (II) mit Verbindungen der Formel (III) beschriebenen Bedingungen, Reduktion der Nitroverbindung und Umsetzung mit einer Verbindung der Formel RCOCl, wie oben beschrieben, herstellen.

Die folgenden Beispiele erläutern die Durchführung der Herstellungsverfahren. Gewichtsteile verhalten sich zu Volumenteilen wie Kilogramm zu Liter.

**Herstellung der Amine der Formel (VI)**

Die Beispiele A und B beziehen sich auf Verbindungen die nicht beansprucht sind; sie dienen lediglich der Illustrierung oder Herstellungsverfahren für die beanspunchten Amine.

**Beispiel A**

16,95 Gewichtsteile 2-Chlorbenzothiazol werden mit 12,5 Gewichtsteilen 4-Mercaptoanilin und 13,8 Gewichtsteilen Kaliumcarbonat zu 150 Volumenteilen Acetonitril 30 Min. unter Rückfluß erhitzt. Das Reaktionsgemisch wird dann abgekühlt, in Eiswasser eingerührt, und abgesaugt. Es werden 24 Gewichtsteile Benzthiazol-2-yl-(4-aminophenyl)-thioether vom Schmelzpunkt 121 bis 123°C erhalten.

**Beispiel B**

16,7 Gewichtsteile 2-Mercaptobenzthiazol werden in 100 Volumenteilen Acetonitril mit 1,38 Gewichtsteilen Kaliumcarbonat und 22,5 Gewichtsteilen 2-Chlor-5-nitrobenzotrifluorid 3 Stunden unter Rückfluß erhitzt. Anschließend kühlt man, gießt das Reaktionsgemisch in Eiswasser und saugt ab. Man erhält 35 Gewichtsteile Benzothiazol-2-yl-(2-trifluormethyl-4-nitrophenyl)-thioether vom Schmelzpunkt 65 bis 67°C.

25,3 Gewichtsteile Benzothiazol-2-yl(2-trifluormethyl-4-nitrophenyl)-thio-ether werden im 100 Volumenteilen Ethanol und 100 Volumenteilen konz. Salzsäure suspendiert und bei 50 bis 60°C portionsweise mit 65 g Zink(II)-chlorid versetzt. Nach dreistündigem Rühren bei 60°C wird in 1000 Volumenteile Eiswasser eingerührt und abgesaugt. Man erhält 24,0 Gewichtsteile 3-Trifluormethyl-4-(benzothiazol-2-ylthio)-anilinhydrochlorid vom Schmelzpunkt 132-134°C (Zersetzung).

Auf analogem Wege lassen sich beispielsweise folgende Amine der Formel (VI) erhalten:

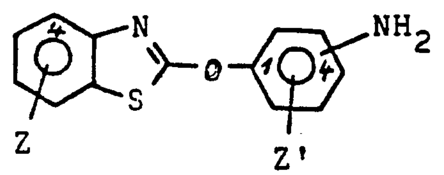

VI

| Z | Z' | Stellung von $NH_2$ | Fp. [°C] |
|---|---|---|---|
| H | H | 3 | 179-181 * |
| H | H | 4 | 211-213 * |
| H | 2-Cl | 4 | 182-184 * |
| H | 2-Br | 4 | |
| H | 2-$CF_3$ | 4 | 189-191 * |
| H | H | 3 | |
| H | H | 4 | |
| 6-Cl | H | 3 | |
| 6-Cl | H | 4 | |
| 6-$CF_3$ | H | 3 | |
| 6-$CF_3$ | H | 4 | |
| 5-Cl | H | 4 | |

| | | |
|---|---|---|
| 5-Cl | H | 3 |
| 5-$CF_3$ | H | 3 |
| 5-$CF_3$ | H | 4 |
| 6-F | H | 3 |
| 6-F | H | 4 |

* als Hydrochlorid isoliert

## Herstellung der Anilinderivate der Formel (I)

**Beispiel 1**

8,48 Gewichtsteile 2-Chlorbenzothiazol werden mit 6,9 Gewichtsteilen Kaliumcarbonat und 9,8 Gewichtsteilen N-(3-Hydroxyphenyl)-N'methoxy-N'methylharnstoff in 150 Volumenteilen Acetonitril 20 Stunden bei Rückfluß gerührt. Anschließend wird gekühlt, in Eiswasser eingerührt und abgesaugt. Man erhält 14,8 Gewichtsteile N-[3-(Benzothiazol-2-yloxy)-phenyl]-N'-methoxy-N'-methylharnstoff vom Schmelzpunkt 90 bis 92°C.

Analog lassen sich beispielsweise folgende Anilinderivate der Formel (I) herstellen:

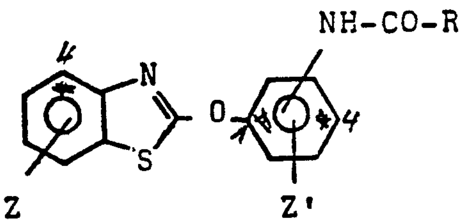

| Nr. | Z | Z' | R | Stellung von -NHCOR | Fp. [°C] |
|---|---|---|---|---|---|
| 1 | H | H | $N{<}^{OCH_3}_{CH_3}$ | 4 | 108-110 |
| 2 | H | H | $N{<}^{CH_3}_{CH_3}$ | 3 | 154-157 |
| 3 | H | H | $N{<}^{OCH_3}_{CH_3}$ | 3 | 89-92 |
| 4 | H | 2-Cl | $N{<}^{OCH_3}_{CH_3}$ | 4 | 111-113 |
| 5 | H | 2-$CF_3$ | $N{<}^{OCH_3}_{CH_3}$ | 4 | 110-112 |
| 6 | H | 2-Br | $N{<}^{OCH_3}_{CH_3}$ | 4 | 135-139 |
| 7 | H | H | $OCH_3$ | 4 | 125-127 |
| 8 | H | H | $SCH_3$ | 4 | 148-150 |
| 9 | H | H | $C_2H_5$ | 4 | 143-145 |
| 10 | H | H | $C_3H_7$ | 4 | |
| 11 | H | H | Cyclopropyl | 4 | 173-175 |
| 12 | H | H | $C_2H_5$ | 3 | 165-167 |
| 13 | H | 2-Cl | $C_2H_5$ | 4 | 139-142 |

| Nr. | Z | Z' | R | Stellung von -NHCOR | Fp. [°C] |
|---|---|---|---|---|---|
| 14 | H | H | 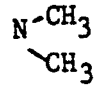 | 4 | 152-155 |
| 15 | 6-Cl | H | 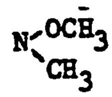 | 3 | |
| 16 | 6-Cl | H | 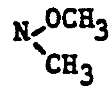 | 4 | 151-153 |
| 17 | 6-Cl | H | $C_2H_5$ | 4 | |
| 18 | $6-CF_3$ | H | 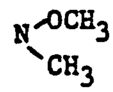 | 4 | |
| 19 | $6-CF_3$ | H | 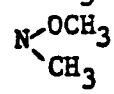 | 3 | |
| 20 | $6-CF_3$ | H | $C_2H_5$ | 4 | |
| 21 | 5-Cl | H | 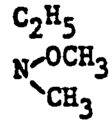 | 3 | |
| 22 | 5-Cl | H | 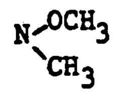 | 4 | 116-118 |
| 23 | 5-Cl | H | $C_2H_5$ | 4 | |
| 24 | 6-F | H | 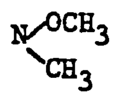 | 4 | |
| 25 | 6-F | H | 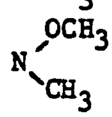 | 3 | |
| 26 | 6-F | H | $C_2H_5$ | 4 | |
| 27 | $5-CF_3$ | H | 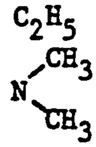 | 4 | |
| 28 | $5-CF_3$ | H | 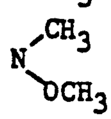 | 3 | |
| 29 | $5-CF_3$ | H | 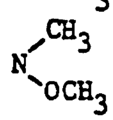 | 4 | 149-152 |
| 30 | H | H | tert.-$C_4H_9$ | 4 | |
| 31 | H | H | sec.-$C_5H_{11}$ | 4 | |
| 32 | H | H | $OCH_3$ | 3 | 161-163 |
| 33 | H | H | $SCH_3$ | 3 | 174-176 |
| 34 | H | H | Cyclopropyl | 3 | |

Die Anilinderivate der Formel (I) können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Gießen, Stäuben, Streuen oder Sprühen auf die Pflanzen oder auf den Boden oder durch Einbringen in das Bewässerungswasser angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 4, werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 6 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr.14 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 20 Teile der Verbindung Nr. 22 werden mit 2 Teilen Calciumsalz der Dodecylbenzolsulfonsäure, 8 Teilen

Fettalkohol-polyglykolether, 2 Teilen Natriumsalz eines Phenol-Harnstoff-Formaldehyd-Kondensates und 68 Teilen eines paraffinischen Mineralöls immig vermischt. Man erhält eine stabile ölige Dispersion.

Die Applikation der Wirkstoffe bzw. der Mittel kann im Vorauflaufverfahren oder im Nachauflaufverfahren erfolgen. Vorzugsweise werden die neuen Wirkstoffe nach dem Auflaufen der unerwünschten Pflanzen ausgebracht. Sind die Wirkstofie für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,1 bis 5 kg Wirkstoff/ha, vorzugsweise 0,25 bis 3,0 kg Wirkstoff/ha.

Der Einfluß von Anilinderivaten der Formel (I) auf das Wachstums von erwünschten und unerwünschten Pflanzen wird anhand von Gewächshausversuchen gezeigt:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät.

Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmenge beträgt 3,0 kg Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff; sie betragen beispielsweise 0,25, 0,5 und 3,0 kg Wirkstoff/ha. Eine Abdeckung unterbleibt bei der Nachauflaufbehandlung.

Die in den Gewächshausversuchen verwendeten Pflanzen setzen sich aus folgenden Arten zusammen:

Abutilon theophrasti (Chinesischer Hanf), Amaranthus spp. (Fuchsschwanz), Chenopodium album (Weißer Gänsefuß), Gossypium hirsutum (Baumwolle), Centaurea cyanus (Kornblume), Ipomoea spp. (Prunkwindearten), Lamium spp. (Taubnessel), Lolium multiflorum (Ital. Raygras), Mercurialis annua (Bingelkraut), Sesbania exaltata (Turibaum), Setaria italica (Kolbenhirse), Sinapis alba (Weißer Senf), Solanum nigrum (Schwarzer Nachtschatten), Triticum aestivum (Weizen).

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckte sich über 2 bis 4 Wochen. Während dieser Zeit werden die Pflanzen gepflegt und ihre Reaktion auf die einzelnen Behandlungen ausgewertet. Bewertet wird nach einer Skala von 0 bis 100. Dabei bedeutet 100 kein Aufgang bzw. völlige Zerstörung zumindest der oberirdischen Sproßteile.

Bei Vorauflaufanwendung von 3,0 kg/ha Wirkstoff zeigen beispielsweise die Verbindungen Nr. 2 und 5 eine gute herbizide aktivität an Lolium multiflorum.

Bei Nachauflaufanwendung und einer Aufwandmenge von 3,0 kg Wirkstoff/ha bekämpfen beispielsweise die Verbindungen Nr. 3, 4, 5, 6, 14, 22 und 30 breitblättrige unerwünschte Pflanzen. In diesen Versuchen zeigt beispielsweise die Verbindung Nr. 1 eine selektive herbizide Wirkung in den Kulturen Baumwolle und Weizen.

In Anbetracht der Verträglichkeit und der Vielseitigkeit der Applikationsmethoden können die erfindungsgemäßen Verbindungen in einer großen Zahl von Kulturpflanzen zur Beseitigung unerwünschter Pflanzen eingesetzt werden.

Zur Verbreiterung des Wirkungsspektrums und auch zur Erzielung synergistischer Effekte können die Anilinderivate der Formel I mit zahlreichen anderen herbiziden Wirkstoffen gemischt und gemeinsam ausgebracht werden. Beispielsweise kommen als Mischungspartner Diazine, 4 H-3,1-Benzoxazin-derivate, Benzothiadiazinone, 2,6-Dinitroaniline, N-Phenylcarbamate, Thiolcarbamate, Halogencarbonsäuren, Triazine, Amide, andere Harnstoffe, Diphenylether, Triazinone, Uracile, Benzofuranderivate, Cyclohexan-1,3-dionderivate und andere in Betracht.

Außerdem kann es von Nutzen sein, die Verbindungen der Formel (I) allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. Anilinderivate der Formel (I)

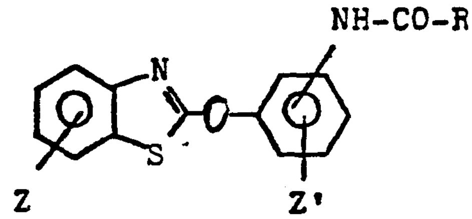

in der
Z Wasserstoff $C_1$-$C_4$-Halogenalkyl oder Halogen,
Z' Wasserstoff, Halogen oder Trifluormethyl
und
R $C_1$-$C_5$-Alkyl, $C_1$-$C_4$-Alkoxy, $C_1$-$C_4$-Alkylthio, gegebenenfalls durch $C_1$-$C_4$-Alkyl substituiertes $C_3$-$C_7$-Cycloalkyl, Di-$C_1$-$C_4$-alkyl-amino, N-$C_1$-$C_4$-Alkyl-H-$C_1$-$C_4$-alkoxyamino bedeuten.

2. Anilinderivate der Formel (I) gemäß Anspruch 1, dadurch gekennzeichnet, daß die Gruppe -NH-CO-R in 4-Stellung steht und R gleich N-$C_1$-$C_4$-Alkyl-N-$C_1$-$C_4$-alkoxyamino bedeutet.

3. Verfahren zur Herstellung der Anilinderivate der Formel (I) gemäß Anspruch 1, dadurch gekennzeichnet, daß man eine Verbindung der Formel (II)

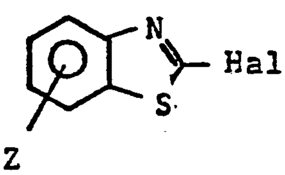

in der Z die im Anspruch 1 genannten Bedeutungen hat und Hal Halogen bedeutet, mit einem Phenol der Formel (III)

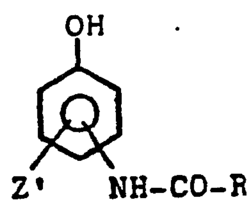

in der Z' und R die im Anspruch 1 genannten Bedeutungen haben, bei einer Temperatur zwischen 20 und 150°C in Gegenwart eines inerten Lösungsmittels und einer Base umsetzt.

4. Verfahren zur Herstellung von Anilinderivaten der Formel (I) gemäß Anspruch 1, dadurch gekennzeichnet, daß man eine Verbindung der Formel (IV)

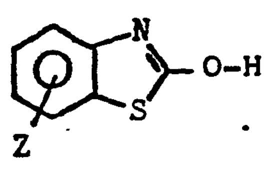

in der Z die im Anspruch 1 genannten Bedeutungen hat, mit einem Halogennitrobenzolderivat der Formel (V)

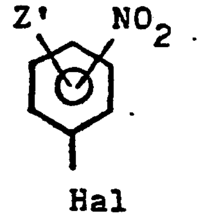

in der Z' die im Anspruch 1 genannten Bedeutungen hat, und Hal für Halogen steht, bei einer Temperatur im Bereich zwischen 20 und 150°C in einem inerten Lösungsmittel in Gegenwart einer Base umsetzt, das so erhaltene Nitroderivat in an sich üblicher Weise zum Amin der Formel (VI)

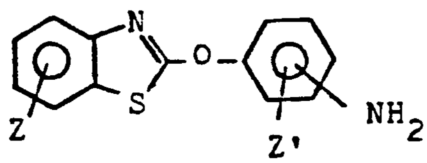

in der Z und Z' die im Anspruch 1 genannten Bedeutungen haben, reduziert und diese mit einer Verbindung der Formel RCOCl, in der R die im Anspruch 1 genannten Bedeutungen hat, bei einer Temperatur zwischen 0 und 50°C in Gegenwart eines inerten Lösungsmittels und einer Base umsetzt.

5. Verfahren zur Herstellung von Anilinderivaten der Formel I gemäß Anspruch 1, dadurch gekennzeichnet, daß man eine Verbindung der Formel (II) gemäß Anspruch 3 mit einem Phenol der Formel (VII)

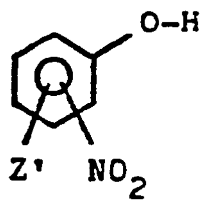

in der

Z' die im Anspruch 1 genannte Bedeutung hat, im Gegenwart eines inerten Lösungsmittels und einer Base bei einer Temperatur zwischen 20 und 150°C umsetzt, das so erhaltene Nitroderivat in an sich üblicher Weise zum Amin reduziert und dieses mit einer Verbindung der Formel RCOCl gemäß Anspruch 4 in Gegenwart eines inerten Lösungsmittels und einer Base umsetzt.

6. Verfahren zur Herstellung von Anilinderivaten der Formel (I) gemäß Anspruch 1, dadurch gekennzeichnet, daß man eine Verbindung der Formel (II) gemäß Anspruch 3 mit einem Phenol der Formel (VIII)

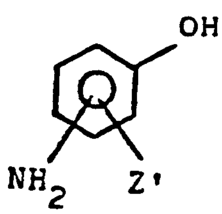

in der

Z' die im Anspruch 1 genannte Bedeutung hat, in Gegenwart eines inerten Lösungsmittels und einer Base bei einer Temperatur zwischen 20 und 150°C umsetzt und die so erhaltene Aminoverbindung durch Umsetzung mit einer Verbindung der Formel RCOCl gemäß Anspruch 4 in ein Anilinderivat der Formel (I) überführt.

7. Herbizid, enthaltend ein Anilinderivat der Formel (I) gemäß Anspruch 1.

8. Verfahren zur Bekämpfung unerwünschten Pflanzenwuchses, dadurch gekennzeichnet, daß man die unerwünschten Pflanzen und/oder die von unerwünschten Pflanzen freizuhaltende Fläche mit einer herbizid wirksamen Menge eines Anilinderivates der Formel (I) gemäß Anspruch 1 behandelt.

9. Amine der Formel (VI)

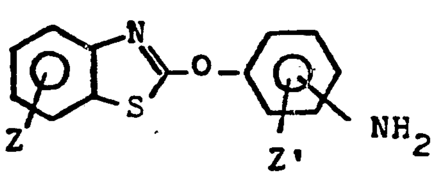

in der Z und Z' die im Anspruch 1 angegebenen Bedeutungen haben.

**Claims**

1. An aniline derivative of the formula (I)

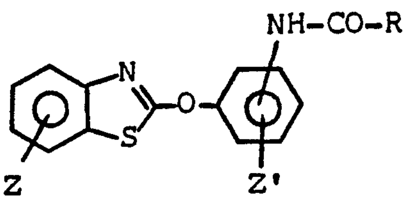

I,

where
Z is hydrogen, $C_1$-$C_4$-haloalkyl or halogen,
Z' is hydrogen, halogen or trifluoromethyl, and
R is $C_1$-$C_5$-alkyl, $C_1$-$C_4$-alkoxy, $C_1$-$C_4$-alkylthio, unsubstituted or $C_1$-$C_4$-alkyl-substituted $C_3$-$C_7$-cycloalkyl, di-$C_1$-$C_4$-alkylamino or N-$C_1$-$C_4$-alkyl-N-$C_1$-$C_4$-alkoxyamino.

2. An aniline derivative of the formula (I) as claimed in claim 1, where the -NH-CO-R group is in the 4-position and R is N-$C_1$-$C_4$-alkyl-N-$C_1$-$C_4$-alkoxyamino.

3. A process for the preparation of an aniline derivative of the formula (I) as claimed in claim 1, wherein a compound of the formula (II)

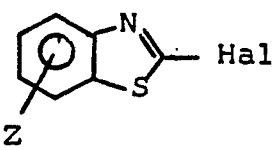

II,

where Z has the meanings given in claim 1, and Hal is halogen, is reacted with a phenol of the formula (III)

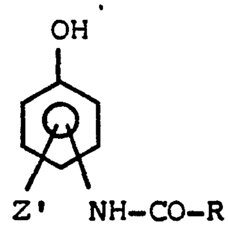

III,

where Z' and R have the meanings given in claim 1, at from 20 to 150°C in the presence of an inert solvent and a base.

4. A process for the preparation of an aniline derivative of the formula (I) as claimed in claim 1, wherein a compound of the formula (IV)

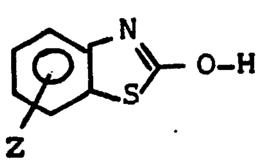

IV,

where Z has the meanings given in claim 1, is reacted with a halonitrobenzene derivative of the formula (V)

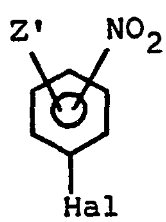

V,

where Z' has the meanings given in claim 1, and Hal is halogen, at from 20 to 150°C in an inert solvent in the presence of a base, the resulting nitro derivative is reduced in a conventional manner to the amine of the formula (VI)

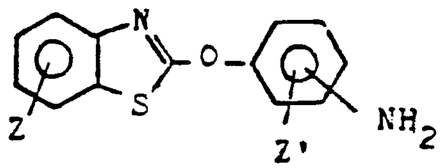

VI,

where Z and Z' have the meanings given in claim 1, and this amine is reacted with a compound of the formula RCOCl, where R has the meanings given in claim 1, at from 0 to 50°C in the presence of an inert solvent and a base.

5. A process for the preparation of an aniline derivative of the formula (I) as claimed in claim 1, wherein a compound of the formula (II) as defined in claim 3 is reacted with a phenol of the formula (VII)

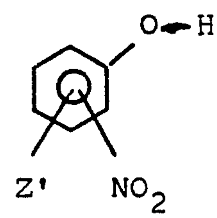

VII,

where Z' has the meanings given in claim 1, at from 20 to 150°C in the presence of an inert solvent and a base, the resulting nitro derivative is reduced in a conventional manner to the amine, and this amine is reacted with a compound of the formula RCOCl as defined in claim 4 in the presence of an inert solvent and a base.

6. A process for the preparation of an aniline derivative of the formula (I) as claimed in claim 1, wherein a compound of the formula (II) as defined in claim 3 is reacted with a phenol of the formula (VIII)

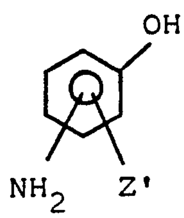

VIII,

where Z' has the meanings given in claim 1, at from 20 to 150°C in the presence of an inert solvent and a base, and the resulting amino compound is converted to an aniline derivative of the formula (I) by reaction with a compound of the formula RCOCl as defined in claim 4.

7. A herbicide containing an aniline derivative of the formula (I) as claimed in claim 1.

8. A process for combating the growth of unwanted plants, wherein the unwanted plants and/or the area to be kept free from unwanted plants are treated with a herbicidally effective amount of an aniline derivative of the formula (I) as claimed in claim 1.

9. An amine of the formula (VI)

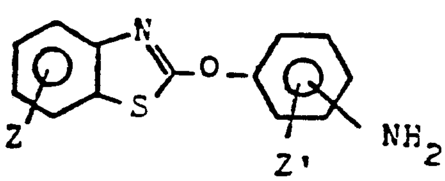

VI,

where Z and Z' have the meanings given in claim 1.

**Revendications**

1. Dérivés de l'aniline de la formule (I)

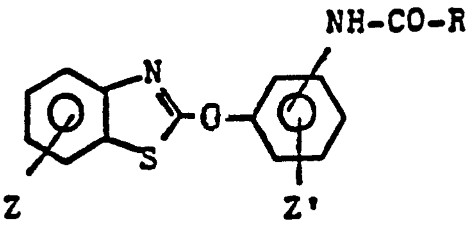

I

dans laquelle
Z désigne un atome d'hydrogène ou d'halogène ou un radical halo-alkyle en $C_1$ à $C_4$,
Z' désigne un atome d'hydrogène ou d'halogène ou un groupe trifluorométhyle et
R représente un radical alkyle en $C_1$ à $C_5$, alcoxy en $C_1$ à $C_4$, alkyl(en $C_1$ à $C_4$)-thio ou un groupe cycloalkyle en $C_3$ à $C_7$ éventuellement alkyl(en $C_1$ à $C_4$)-substitué, di-alkyl(en $C_1$ à $C_4$)-amino ou N-alkyl(en $C_1$ à $C_4$)-N-alcoxy(en $C_1$ à $C_4$)-amino.

2. Dérivés de l'aniline de la formule (I) selon la revendications 1, caractérisés en ce que le groupe -NH-CO-R se trouve dans la position 4 et R = N-alkyl(en $C_1$ à $C_4$)-N-alcoxy(en $C_1$ à $C_4$)-amino.

3. Procédé de préparation de dérivés de l'aniline de la formule (I) selon la revendication 1, caractérisé en ce que l'on fait réagir un composé de la formule (II)

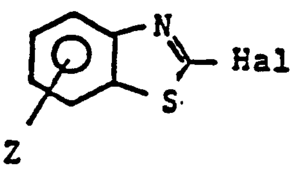

II

dans laquelle Z possède les significations définies dans la revendication 1 et Hal désigne un atome d'halogène, à une température comprise entre 20 et 150°C et en présence d'un solvant inerte et d'une base avec un phénol de la formule (III)

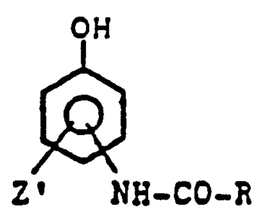

III

dans laquelle Z' et R possèdent les significations définies dans la revendication 1.

4. Procédé de préparation de dérivés de l'aniline de la formule (I) selon la revendication 1, caractérisé en ce que l'on fait réagir un composé de la formule (IV)

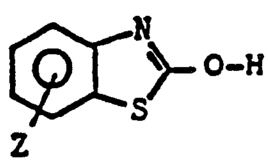

IV

dans laquelle Z possède les significations définies dans la revendication 1, à une température comprise entre 20 et 150°C, dans un solvant inerte et en présence d'une base, avec un dérivé d'halo-nitro-benzène de la formule (V)

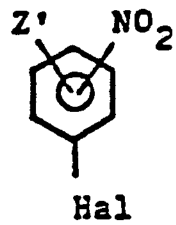

V

dans laquelle Z' possède les significations définies dans la revendication 1 et Hal désigne un atome d'halogène, puis on réduit le dérivé nitro obtenu de façon usuelle en amine de la formule (VI)

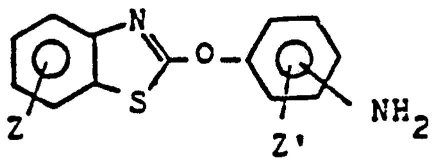

VI

dans laquelle Z et Z' possèdent les significations définies dans la revendication 1, que l'on fait ensuite réagir à une température comprise entre 0 et 50° C et en présence d'un solvant inerte et d'une base avec un composé de la formule RCOCl, dans laquelle R possède les significations définies dans la revendication 1.

5. Procédé de préparation de dérivés de l'aniline de la formule (I) selon la revendication 1, caractérisé en ce que l'on fait réagir un composé de la formule (II) selon la revendication 3 à une température comprise entre 20 et 150° C et en présence d'un solvant inerte et d'une base avec un phénol de la formule (VII)

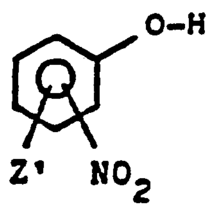

VII

dans laquelle Z' possède la signification définie dans la revendication 1, puis on réduit le dérivé nitro obtenu de façon usuelle en amine, que l'on fait ensuite réagir en présence d'un solvant inerte et d'une base avec un composé de la formule RCOCl selon la revendication 4.

6. Procédé de préparation de dérivés de l'aniline de la formule (I) selon la revendication 1, caractérisé en ce que l'on fait réagir un composé de la formule (II) selon la revendication 3 à une température comprise entre 20 et 150° C et en présence d'un solvant inerte et d'une base avec un phénol de la formule (VIII)

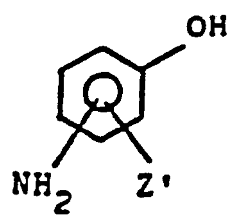

VIII

dans laquelle Z' possède la signification définie dans la revendication 1, puis on transforme le composé amino obtenu, par réaction avec un composé de la formule RCOCl selon la revendication 4, en un dérivé de l'aniline de la formule (I).

7. Composition herbicide, contenant un dérivé de l' aniline de la formule (I) selon la revendication 1.

8. Procédé de lutte contre le développement de plantes indésirables, caractérisé en ce que l'on traite les plantes indésirables et (ou) la surface, sur laquelle doit être empêché le développement de plantes indésirables, avec une quantité efficace du point de vue herbicide d'un dérivé de l'aniline de la formule (I) selon la revendication 1.

9. Amines de la formule (VI)

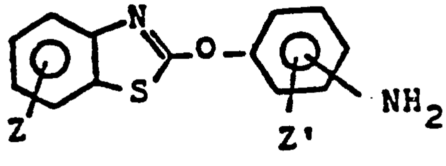

VI

dans laquelle Z et Z' possèdent les significations définies dans la revendication 1.